# EUROPEAN PATENT APPLICATION

(11) **EP 4 155 297 A1**
(43) Date of publication of application: **29.03.2023**
(21) Application number: 21198883.7
(22) Date of filing: 24.09.2021
(51) Int. Cl.: C07D 307/38

(54) **METHOD TO PREPARE FURAN-BASED SURFACTANTS**

(71) Applicant: Nederlandse Organisatie voor Toegepast-Natuurwetenschappelijk Onderzoek TNO, 2595 DA 's-Gravenhage (NL)
(72) Inventor: CROCKATT, Marc, 2595 DA 's-Gravenhage (NL)
(74) Representative: V.O.

(57) **Abstract**

The invention is directed to the preparation of furan-based surfactant or a salt thereof according to any of formula (IVa)-(IVf), wherein R³ is H, OR⁴ or R⁴; R² is H or an aliphatic chain, R⁴, R⁵ and R⁶ independently are an aliphatic chain; and wherein G is a moiety comprising a hydrophilic group. 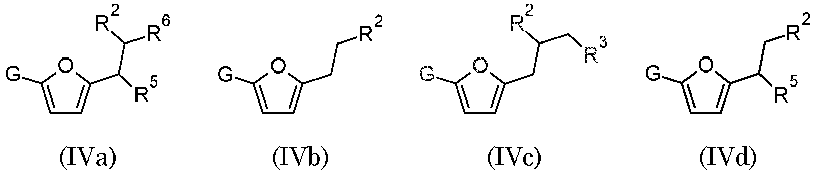 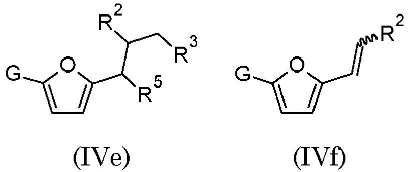 These compounds are accessible through a first furan-based surfactant precursor of formula (I), wherein R¹ is H or CH₂OH, R² is H or an aliphatic chain, and R³ is H, OR⁴ or R⁴, wherein R₄ is H or an aliphatic chain.

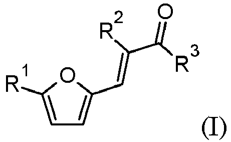

## Description

The invention is in the field of surfactants. In particular, the invention is directed to a method to prepare furan-based surfactants.

Surfactants, or surface-active agents, are chemical compounds that are capable of lowering the surface tension between two liquids or between a gas and a liquid. Surfactants are widely used for a range of applications, such as detergents, emulsifiers, foaming agents and dispersants. Typically, the compounds are amphiphilics, *i*.*e*. compounds with both a hydrophobic tail and a hydrophilic head. This combination may allow for a water-soluble component and a water-insoluble (*i*.*e*. oil-soluble) component. Accordingly, the surfactant may absorb at *i*.*a*. oil/water interphases and can provide interesting properties for a variety of applications.

It is expected that the market for surfactants undergoes continuous growth in the coming years. The main contributor to the market constitutes detergents and cleaners for households as over 50% of all surfactants typically end up in these products. Currently, the largest production comprises petrochemical-based linear alkylbenzene sulfonates (LAS), with around 4 million tons per year being produced and consumed.

LAS surfactants are typically a mixture of compounds based on a hydrophilic sulfonate head attached to a hydrophobic benzene ring which is attached to a hydrophobic alkyl chain. The big advantage of LAS is its tolerance of hard water, which commonly inhibits the functioning of other groups of surfactants. Further, LAS typically has a low critical micelle concentration, low Krafft temperature, fast wetting and good foaming behaviour. LAS is accordingly suitable for broad use in a variety of applications.

However, LAS is fossil-based and has a negative environmental impact. Accordingly, there is a strong desire to provide more sustainable surfactants which have comparable or improved properties compared to LAS.

One method to provide surfactants produced from bio-based chemicals such as sugar-derived furans is described in e.g. WO2017/079718 and by Park et al. (ACS Cent. Sci. 2016, 2, 11, 820-824). Here, oleo-furan surfactants derived from furan and fatty acids are described. By adjusting the fatty acid the surfactant properties could be altered. Additionally, the surfactants could better tolerate the metal ions contained in hard water, therefore making chelating agents, which are typically added to *e.g.* detergents, unnecessary. However, the surfactants are synthesized from furan. While it is a bio-based molecule, it requires decarbonylation of furfural to prepare furan, thereby decreasing the atom efficiency and resulting in a waste stream of carbon monoxide. Furthermore, it requires an additional processing step, resulting in increased cost and inefficiency. Moreover, the process may be challenging to scale-up.

Other bio-based surfactants are described by Kipshagen et al. (Green Chem. 2019, 21, 3882). Here *i*.*a*. furfural and 5-hydroxymethylfurfural (5-HMF) are used as the basis for the surfactants. In the case of furfural, it is converted into tetrahydrofuran followed by side-chain manipulation and the introduction of a hydrophilic head. In the case of HMF, it is first hydrogenated to 2,5-bis(hydroxymethyl)furan (BHMF) followed by side-chain manipulation and introduction of the hydrophilic head.

5-HMF has also been used as a starting material for surfactants as described in WO2016028845, wherein the final surfactants were prepared by esterification, amination and alkylation to produce non-ionic surfactants. However, these do not mimic LAS.

Other surfactants are described in e.g. WO2021/083642, WO2015/084813 and WO2017/079719.

It is an object of the present inventors to provide an improved method to prepare furan-based surfactants that overcomes at least part of the above-mentioned drawbacks. The present inventors realized that this can be achieved by a process comprising a reaction of a furfural compound and an aldehyde, ester or ketone. It was found that this approach advantageously provides access to surfactants with a variety of hydrophobic tails and hydrophilic heads, enabling fine-tuning of the furan-based surfactant specific to the particular application of the surfactant. The method according to the present invention is further advantageously efficient and suitable to be scaled-up.

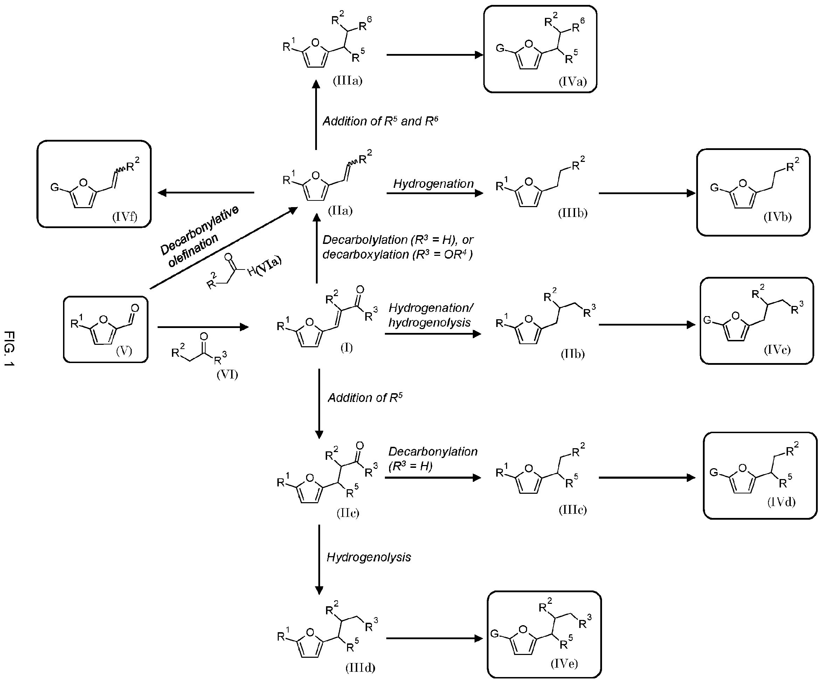

Figure 1 illustrates a schematic overview of the possible reaction paths according to preferred embodiments of the present invention.

The present invention thus advantageously provides access to furan-based surfactants or salts thereof according to any of formula (IVa)-(IVf), wherein R³ is H, OR⁴ or R⁴; R², R⁴, R⁵ and R⁶ independently are an aliphatic group, preferably an aliphatic chain, such that they together form a hydrophobic tail comprising six or more carbon atoms attached to the furan moiety; and wherein G is a moiety comprising a hydrophilic group.

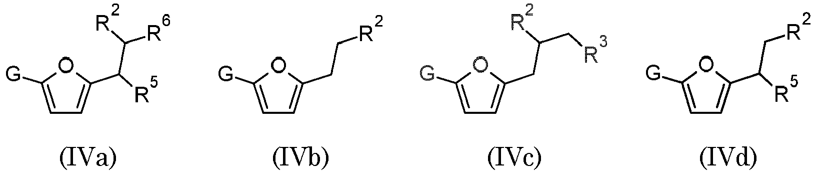

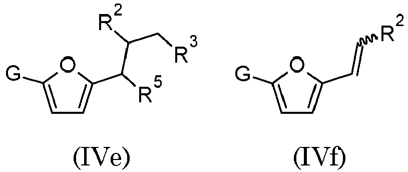

The groups R², R⁴, R⁵ and R⁶ form together with the carbon atoms with which they are attached to the furan moiety the hydrophobic tail. As such, these groups, whichever may be present, comprise 6 or more carbon atoms, preferably 6 to 26 carbon atoms, most preferably between 6 and 18 carbon atoms together with the carbon atoms with which they are attached to the furan moiety. In other words, the substituents ―CH(R⁵)-CH(R²)(R⁶), - CH₂-CH₂R², ―CH₂-CH(R²)-CH₂R³, ―CH(R⁵)-CH₂(R²), ―CH(R⁵)-CH(R²)-CH₂R³, and ―CH=CHR² of formulae (IVa)-(IV)f respectively, each represent the hydrophobic tail comprising 6 or more carbon atoms, preferably 6 to 26 carbon atoms, most preferably between 6 and 18 carbon atoms. Thus, the present invention advantageously provides good control over the substitution pattern of the hydrophobic chain.

As shown in the overview illustrated in Figure 1, these compounds are all accessible from furfural or HMF through a first furan-based surfactant precursor of formula (I), wherein R¹ is H or CH₂OH, R² is H or an aliphatic group, preferably an aliphatic chain, and R³ is H, OR⁴ or R⁴, wherein R₄ is H, an aliphatic group, or an aliphatic chain. 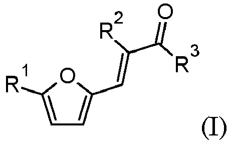 Thus, in a first aspect, the invention is directed to a method for the preparation of a furan-based surfactant precursor (I) according to formula (I) or a salt thereof.

More specifically, the invention is directed to a method for the preparation of a first furan-based surfactant precursor of formula (I), said method comprising reacting a furanic compound according to formula (V) with a carbonyl compound according to formula (VI), 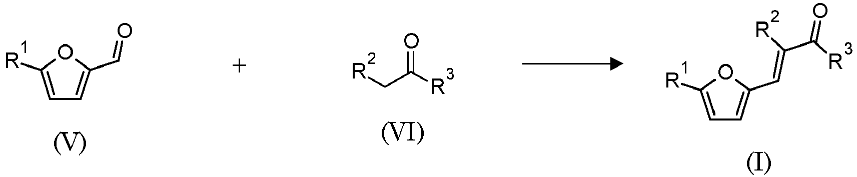
wherein R¹ is H or CH₂OH;
R² is H or an aliphatic group, preferably an aliphatic chain; and
R³ is H, OR⁴ or R⁴, wherein R₄ is H, an aliphatic group or an aliphatic chain.

Aliphatic group is herein used to describe aliphatic groups comprising one or more carbon atoms, e.g. methyl, ethyl, propyl and the like. The aliphatic group may be an aliphatic chain. Aliphatic chain is herein used to describe a carbon chain comprising multiple carbon atoms that may be linear, branched, or cyclic, saturated or unsaturated but not aromatic. Thus, the carbons in the aliphatic chain may be joined by single bonds, one or more carbon pairs may be joined by a double bond and/or one or more carbons may be joined by a triple bond. Also, the chain may comprise one or more non-aromatic carbocyclic moieties. In case the aliphatic chain is branched or cyclic, the main chain (*i*.*e*. the longest chain of carbon atoms) is considered the backbone chain. The hydrophilic tail may suitably be an aliphatic hydrocarbon chain or an aliphatic ether. Preferably, if the aliphatic chain comprises a certain number of carbon atoms, e.g. at least 6 carbon atoms, it is preferred that the backbone chain comprises said number of carbon atoms, e.g. at least 6 carbon atoms. The branch may on itself also be an aliphatic chain.

The terms polar, apolar, hydrophilic and hydrophobic are commonly used in the field of surfactants. It is commonly known that a surfactant is amphiphilic and comprises hydrophobic (or apolar) and hydrophilic (or polar) moieties. Accordingly, if a compound in accordance with the present invention acts as a surfactant, the moiety of the substituents G of the compound are considered to be hydrophilic, while the aliphatic chain is considered hydrophobic. In general, hydrophilic is used to describe the capacity of a molecular entity or of a substituent to interact with polar solvents, in particular with water, or with other polar groups and the tendency to mix, be wetted and/or dissolve in water. Typically, hydrophobic is used for moieties that form Van der Waals bonds and minimal to no hydrogen bonds. For instance, aliphatic chains may be considered hydrophobic, while sulfonic and carboxylic acid groups are considered hydrophilic.

In the field of surfactants, various polar head groups are known. The hydrophilic moiety may thus be an ionic moiety, e.g. an anionic moiety, a cationic, or a non-ionic moiety, in accordance with conventionally known ionic and non-ionic surfactants. Accordingly, suitably, G may independently comprise anionic moieties such as sulfate, sulfonate, sulfinate, thiosulfate, carboxylate, phosphate, phosphonate and the like.

Examples of suitable non-ionic moieties that G may comprise include poly(ethylene oxide), poly(co-ethylene oxide co-propylene oxide) (also referred to as poloxamers), polyglycosides, isosorbide and its derivatives, 1,4-sorbitane and its derivatives, and the like.

The furanic compound may for instance be derived from a C₅-sugar, such as furfural, or from a C_{6-sugar}, such as HMF. The present inventors surprisingly found that furfural can particularly beneficially be used in the present method. Using furfural as a starting material increases the atom efficiency as there is no decarbonylation step involved, in contrast to furan. Additionally, furfural is typically more stable and easier to purify than 5-HMF. Accordingly, the furanic moiety is preferably furfural.

Further, advantageously, furfural is a readily available furanic compound as it is for instance currently commercially produced from hemicellulose waste streams from the agricultural industry (e.g. corn, sugar). The furfural is preferably bio-based.

The method according to one aspect of the invention comprises a reaction of the furanic compound of formula (V) with the carbonyl compound of formula (VI). Such a reaction is under certain conditions known as an aldol reaction. It may be appreciated that the aldehyde and/or ketone can also be formed *in situ* by oxidation of an alcohol and/or reduction of a carboxylic acid or derivative thereof. For example, in an embodiment wherein R³ is H, compound (VI) may be *in situ* prepared by oxidizing R²(CH₂)₂OH or by reducing R²CH₂CO₂R⁴.

Alcohols and/or carboxylic acids may be preferred in order to *in situ* or *ex situ* prepare the carbonyl compound of formula (VI) as these can be bio-based. Aldol reactions are well-known in the art. The carbonyl compound of formula (VI) preferably comprises at least one hydrophobic tail. The hydrophobic tail may suitably be an aliphatic hydrocarbon chain or an aliphatic ether. For instance, the carbonyl compound of formula (VI) can be based on a fatty acid. The hydrophobic tail preferably comprises an aliphatic chain of preferably at least 6 carbon atoms in the backbone chain.

In a particularly preferred embodiment, the method according to one aspect of the invention is carried out using furfural and an aldehyde comprising a hydrophobic tail to form an alpha-beta-unsaturated aldehyde in accordance with formula (Ia).

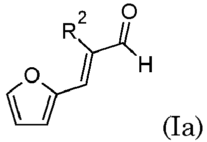

The reaction using furfural or HMF is favorable over the reaction described in the aforementioned article by Park *et al*. as the present method involves the use of an aldehyde as reagent, instead of an anhydride. This beneficially reduces the number of processing steps and may allow for reduced cost, time and the number of equivalents of feedstock required (*i*.*e*. improve the atom-efficiency). Additionally, there is no need for trifluoroacetic anhydride (TFAA), which is an expensive reagent and required in equimolar amounts.

By amending the length and composition of the hydrophobic tail, the biodegradability, solubility and surfactant properties may be altered. Surfactant properties may include critical micelle concentration, Krafft temperature, Draves wetting, foam growth rate, foam height and micelle stability. Depending on the final application, a more hydrophobic or more hydrophilic surfactant may be desirable. For instance, a longer alkyl chain would typically result in an increased hydrophobicity. This potential tuning of the surfactant properties can also be seen in the article by Park *et al*.

The surfactant precursor according to formula (I) can be further subjected to a variety of transformations to the surfactants according to any of formulae (IVa)-(IVd). An overview of the reaction paths can be seen in Figure 1. The basic principles behind these reactions are in general known in the art and can for instance be categorized as decarbonylation, hydrogenolysis, hydrogenation and/or addition reactions.

Accordingly, in a further aspect, the present invention is directed to a method for the preparation of a second furan-based surfactant precursor of any of formulae (IIa)-(IIc), said method comprising reacting the first furan-based surfactant precursor of formula (I) in, respectively, a decarbonylation reaction wherein R³ is H or decarboxylation reaction wherein R³ = OR⁴), a hydrogenation and hydrogenolysis, or an addition reaction, 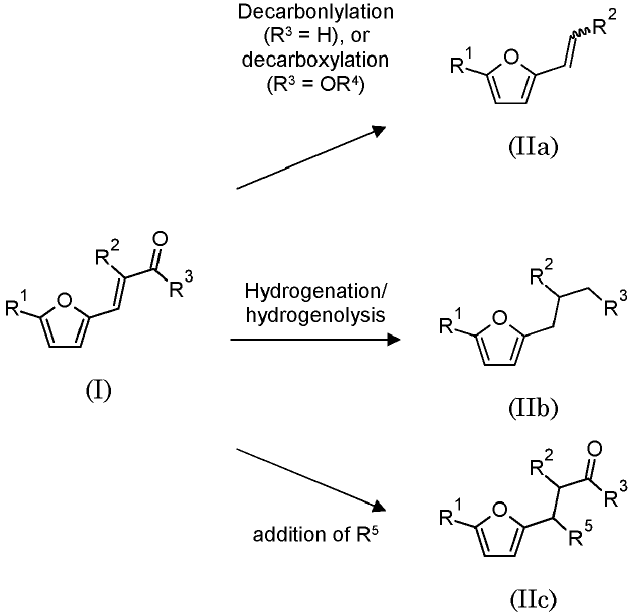 In formulae (I), (IIa)-(IIc), R¹ is H or CH₂OH; R² is H or an aliphatic group, preferably an aliphatic chain; R³ is H, OR⁴ or R⁴, wherein R₄ is H or an aliphatic group, preferably an aliphatic chain; and R⁵ is an aliphatic group, preferably an aliphatic chain.

Decarbonylation is a reaction that involves the loss of CO from an aldehyde. The decarbonylation can be performed in the presence of a catalyst, such as a soluble metal complex. Decarbonylation may be the reaction to be performed on the surfactant precursor (I) and it can result in intermediate (IIa).

Decarboxylation is a reaction that involves the loss of CO₂ from a carboxylic acid. The decarboxylation can be performed in the presence of a catalyst, such as a soluble metal complex. The carboxylic acid (*i*.*e*. the compound of formula (I) wherein R³ = OH) can be obtained directly from the reaction of (V) and (VI) when R³ = OH or the carboxylic acid can be obtained *in situ* in the decarboxylation reaction by a hydrolysis of the compound of formula (I) wherein R³ = OR⁴, wherein R⁴ is an aliphatic chain. Decarbonylation may be the reaction to be performed on the surfactant precursor (I) and it can result in intermediate (IIa).

It may be preferred to combine the aldol reaction and decarbonylation to obtain the second furan-based surfactant precursor (IIa). Such a reaction may be referred to as a decarbonylative olefination as described by *e.g.* Ainembabazi et al. (J. Am. Chem. Soc. 2020, 142, 2, 696-699). Decarbonylative olefination can be considered as a more atom-economical alternative to a conventional Wittig reaction. For decarbonylative olefination, a catalyst such as a palladium (Pd) catalyst is typically required. Advantageously, such a reaction results in less waste and reduces processing inefficiencies when compared to the separate two-step synthesis and the conventional Wittig reaction.

Accordingly, another aspect of the present invention is directed to the preparation of the first furan-based surfactant precursor of formula (IIa), said method comprising reacting a furanic compound according to formula (V) with a carbonyl compound according to formula (VIa) (*i*.*e*. the carbonyl compound of formula (VI) wherein R³ is H) in a decarbonylative olefination reaction, according to the following scheme. 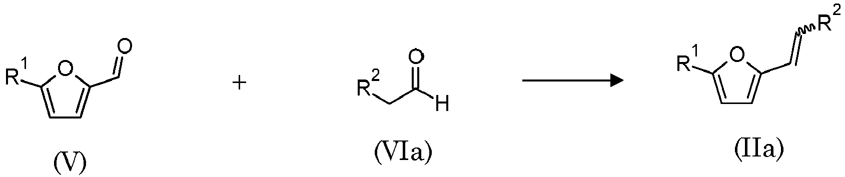 In formulae (VIa) and (IIa), R¹ and R² are as defined for formula (VI) and (II). Formula IIa represents both regioisomers (E- and Z-conformation on the double bond), as represented by 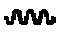 R².

The first furan-based surfactant precursor may further be subjected to hydrogenolysis and hydrogenation to obtain the second furan-based surfactant precursor of formula (IIb). Both hydrogenolysis and hydrogenation are generally carried out with hydrogen in the presence of a catalyst, such as a catalyst surface comprising palladium, nickel and/or platinum.

In another embodiment, the first furan-based surfactant precursor is reacted in an addition reaction to form the second furan-based surfactant precursor of formula (IIc). The addition reaction typically comprises a conjugate addition, also referred to as a 1,4-addition. Such reactions are commonly known in the art and refer to nucleophilic addition directed to the electrophilic carbon of a C=C in an alpha-beta unsaturated system. The reactant introducing R⁵ may be any suitable reactant to introduce an aliphatic chain. The second furan-based surfactant precursor of formula (IIb) and (IIc) may be any stereoisomer or a mixture of one or more stereoisomers. Dependent on the nucleophile, R₅ is an addition moiety. The addition moiety can be, but is not limited to a halogen, amine group, thiol group, or an aliphatic chain. It may be appreciated that the skilled person is aware of many possible moieties that are a result of an addition reaction to an alpha-beta unsaturated system.

It may be appreciated that the second furan-based surfactant precursors can be prepared from a first furan-based surfactant precursor that is obtained according to the reaction of the furanic compound of formula (V) with the carbonyl compound of formula (VI), but that the first furan-based surfactant precursors may also be provided otherwise.

In particular embodiments, the second furan-based surfactant precursors are directly converted to surfactant compound, e.g. (IIb) to (IVc) and (IIa) to (IVa). However, it may be preferred to introduce more aliphatic chains and/or to take out a compound's functionality (e.g. reduce a double or triple bond).

Accordingly, a further aspect of the present invention is directed to the preparation of a third-generation furan-based surfactant precursor, *i.e.* a third furan-based surfactant precursor of any of formulae (IIIa)-(IIId). Said method comprises reacting the second furan-based surfactant precursor of formula (IIa), (IIa), (IIc) wherein R³ is H and (IIc), respectively, in an addition reaction, a hydrogenation reaction, a decarbonylation reaction, or a hydrogenolysis reaction, respectively. 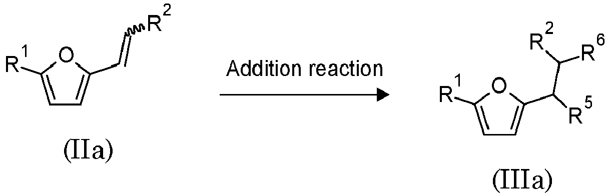 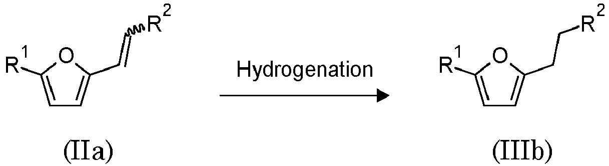 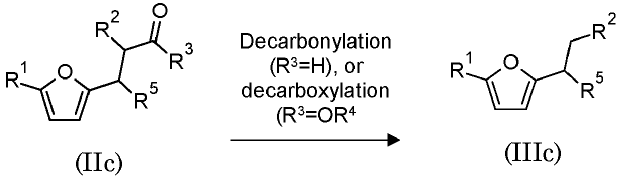 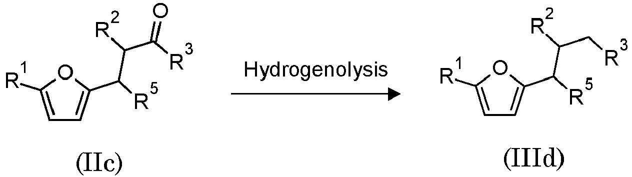 In formulae (IIa)-(IIc) and (IIIa)-(IIId), R¹ is H or CH₂OH; R² is H or an aliphatic group, preferably an aliphatic chain; and R⁵ and R⁶ are independently H or an aliphatic group, preferably an aliphatic chain. As for formulae (IVa)-(IVf), the groups R², R⁴, R⁵ and R⁶ form together with the carbon atoms with which they are attached to the furan moiety the hydrophobic tail. As such, these groups, whichever may be present, comprise 6 or more carbon atoms, preferably 6 to 26 carbon atoms, most preferably between 6 and 18 carbon atoms together with the carbon atoms with which they are attached to the furan moiety. In other words, the substituents - CH(R⁵)-CH(R²)(R⁶), ―CH₂-CH₂R², ―CH(R⁵)-CH₂(R²), and ―CH(R⁵)-CH(R²)-CH₂R³ of formulae (IIIa)-(IIId) respectively, each represent the hydrophobic tail comprising 6 or more carbon atoms, preferably 6 to 26 carbon atoms, most preferably between 6 and 18 carbon atoms.

It may be appreciated that the third furan-based surfactant precursors can be prepared from a second furan-based surfactant precursor that is obtained according to the reaction of the first furan-based surfactant precursor of formula (I) as described herein, but that the second furan-based surfactant precursors may also be provided otherwise.

The addition reaction of the second furan-based surfactant precursor of formula (IIa) to form the third furan-based surfactant precursor of formula (IIIa) can be a commonly known electrophilic addition reaction, metal-catalyzed alkene addition and the like. Examples thereof include the Heck reaction and the Suzuki reaction.

As described for the second furan-based surfactant precursor above, the decarbonylation can be performed in the presence of a catalyst, such as a soluble metal complex. Similarly, the hydrogenation and the hydrogenolysis of the second furan-based surfactant precursor of formulae (IIa) and (IIc) to form the third furan-based surfactant precursor of formulae (IIIb) and (IIId), respectively, can also generally be carried out with hydrogen in the presence of a catalyst, such as a catalyst surface comprising palladium, nickel and/or platinum.

The third furan-based surfactant precursor of formula (IIIa), (IIIc) and (IIId) may be any stereoisomer or a mixture of one or more stereoisomers.

The first, second and third surfactant precursors can be used to prepare furan-based surfactants of formulae (IVa)-(IVf) or salts thereof. Such furan-based surfactants can also function as chelating agents, typically reducing or completely removing the need to add any additional chelating agents to a formulation such as a detergent.

Accordingly, a further aspect of the present invention is directed to a method for the preparation of a furan-based surfactant or a salt thereof according to any of formula (IVa)-(IVf), said method comprising reacting the first or second furan-based surfactant precursor of any of formulae (IIIa), (IIIb), (IIb), (IIIc), (IIId) and (IIa) respectively, to introduce the hydrophilic group G, 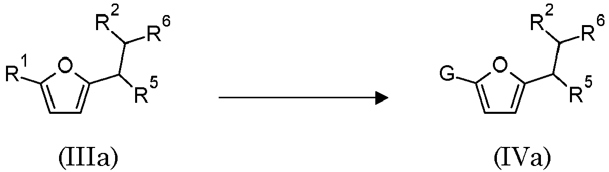 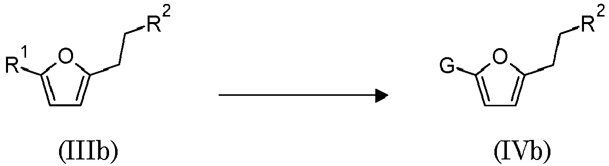 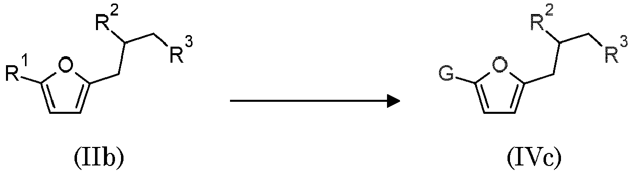 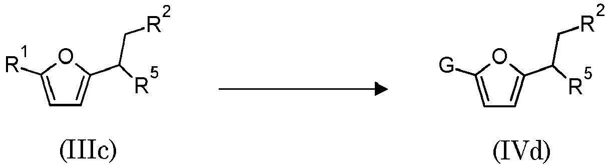 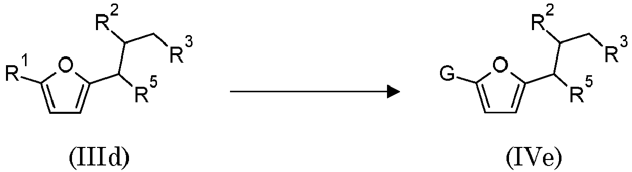 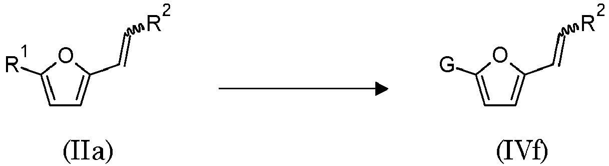 In formula (IIIa), (IIIb), (IIb), (IIIc), (IIId) (IIa) and (IVa)-(IVf), R¹ is H or CH₂OH; R³ is H, OR⁴ or R⁴; R² is H or an aliphatic group, preferably an aliphatic chain, R⁴, R⁵ and R⁶ are independently an aliphatic groups, preferably an aliphatic chain; and G is a moiety comprising a hydrophilic group. The groups R², R⁴, R⁵ and R⁶ form together with the carbon atoms with which they are attached to the furan moiety the hydrophobic tail. As such, these groups, whichever may be present, comprise 6 or more carbon atoms, preferably 6 to 26 carbon atoms, most preferably between 6 and 18 carbon atoms together with the carbon atoms with which they are attached to the furan moiety. In other words, the substituents ―CH(R⁵)-CH(R²)(R⁶), - CH₂-CH₂R², ―CH₂-CH(R²)-CH₂R³, ―CH(R⁵)-CH₂R², ―CH(R⁵)-CH(R²)-CH₂R³, and ―CH=CHR² of formulae (IIIa), (IIb), (IIIc), (IIId) and (IIa) respectively, as well as of formulae (IVa)-(IVf) respectively, each represent the hydrophobic tail comprising 6 or more carbon atoms, preferably 6 to 26 carbon atoms, most preferably between 6 and 18 carbon atoms.

The furan-based surfactants of formula (IVa), (IVc), (IVd) and (IVe) may be any stereoisomer or a mixture of one or more stereoisomers. Formula (IVf) represents both regioisomers (E- and Z-conformation on the double bond), as represented by 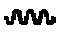 R².

It may be appreciated that the furan-based surfactant or a salt thereof can be prepared from a third or a second furan-based surfactant precursor that is directly obtained according to the reaction of the first and second furan-based surfactant precursors as described herein, but that the second or third furan-based surfactant precursors may also be provided otherwise.

In formulae (IVa)-(IVf), G is a moiety comprising a hydrophilic group. In particular embodiments wherein furfural is used as a starting material, G may represent the hydrophilic group. In particular embodiments wherein HMF is used, G may represent a -CH₂-hydrophilic group.

The hydrophilic moiety may thus be an ionic moiety, e.g. an anionic, a cationic, or a non-ionic moiety. Examples of suitable non-ionic moieties that G may comprise include poly(ethylene oxide), poly(co-ethylene oxide co-propylene oxide) (also referred to as poloxamers), polyglycosides, isosorbide and its derivatives, 1,4-sorbitane and its derivatives, and the like. These moieties can readily be introduced by a reaction of the hydroxyl group of the second and third precursors wherein R¹ is CH₂OH.

The hydrophilic group is preferably an ionic group and can be present with a counter-ion to balance the charges. It may be appreciated that the counter-ions can be any ion that balances the charge, for instance, if the hydrophilic group has a monovalent negative charge, the counter-ion can be e.g. sodium (Na⁺), potassium (K⁺), lithium (Li⁺) and/or ammonium (NH₄⁺). Preferably, the counter-ion is an ammonium ion, an alkali metal ion or alkaline earth metal ion. Suitable hydrophilic groups and counter-ions are for instance described in WO2017/079719.

The hydrophilic group, and preferably G as such, is preferably selected from the group consisting of sulfate (-O-SO₃⁻), sulfonate (-SO₃⁻), sulfinate (-SO₂⁻), thiosulfate (-O-S₂O₂⁻), sulfamidate (-NH-SO₃⁻), carboxylate (-CO₂⁻), sarcosinate and taurate (-NR-R-CO₂⁻), phosphate (-O-PO₃⁻ or -O-PO₂-OR⁻), pyrophosphate (-O-PO₂-O-PO₂-OR²⁻), phosphonate (-PO₂R⁻ or - PO₃⁻), amines or ammonium (-NR₃⁺), polyammonium (-NR₂-R-NR₃²⁺), hydroxyammonium (-NR₂OH⁺), pyridinium (-NC₅H₅⁺), picolinium (-NC₅H₅-R⁺), imidazolinium 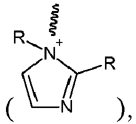 benzimidazolinium 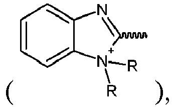 oxonium (-OR₂⁺), sulfonium (-SR₂⁺) and phosphonium (-PR₃⁺). Herein 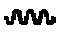 represents the bond to the furanic ring and R is H or a C₁-C₃ alkyl. Particularly good surfactant properties are obtained for sulfonate as hydrophilic group.

The method to prepare the furan-based surfactant comprises introducing the hydrophilic group to the appropriate second or third precursor. The skilled person can suitable select appropriate reactions to introduce the hydrophilic group. For example, sulfonate as the hydrophilic group may be introduced by reacting the appropriate second or third precursor with sulfur trioxide. This is a particular could method for those second or third precursor wherein R¹ is H.

In a particular embodiment, the hydroxyl of the second and/or third precursors wherein R¹ is CH₂OH can be substituted by a hydrophilic group. In such embodiments, G may be selected from the group consisting of methylene sulfate (-CH₂O-SO₃⁻), methylene sulfonate (-CH₂SO₃⁻), methylene sulfinate (-CH₂SO₂⁻), methylene thiosulfate (-CH₂O-S₂O₂⁻), methylene sulfamidate (-CH₂NH-SO₃⁻), methylene carboxylate (-CH₂CO₂⁻), methylene sarcosinate and taurate (-CH₂NR-R-CO₂⁻), methylene phosphate (-CH₂O-PO₃⁻ or - CH₂O-PO₂-OR⁻), methylene pyrophosphate (-CH₂O-PO₂-O-PO₂-OR²⁻ ), methylene phosphonate (-CH₂PO₂R⁻ or - CH₂PO₃⁻), methylene amines or ammonium (-CH₂NR₃⁺), methylene polyammonium (-CH₂NR₂-R-NR₃²⁺), methylene hydroxyammonium (-CH₂NR₂OH⁺), methylene pyridinium (-CH₂NC₅H₅⁺), methylene picolinium (-CH₂NC₅H₅-R⁺), methylene imidazolinium 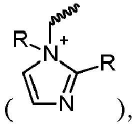 methylene benzimidazolinium 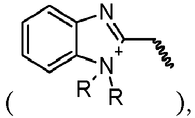 methylene oxonium (-CH₂OR₂⁺), methylene sulfonium (-CH₂SR₂⁺) and methylene phosphonium (-CH₂PR₃⁺), wherein R is H or a C₁-C₃ alkyl, preferably H or Me.

In a particularly preferred embodiment, furfural and an aldehyde of a fatty acid are used as a starting material for the surfactant precursor and a sulfonate is introduced as hydrophilic group. Accordingly, in particular embodiments, the full process from furfural to the furan-based surfactant is according to any of Schemes 1-7.

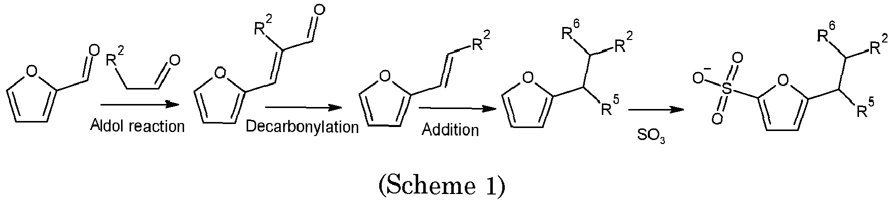

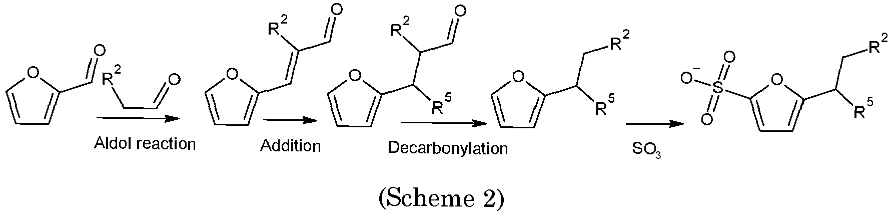

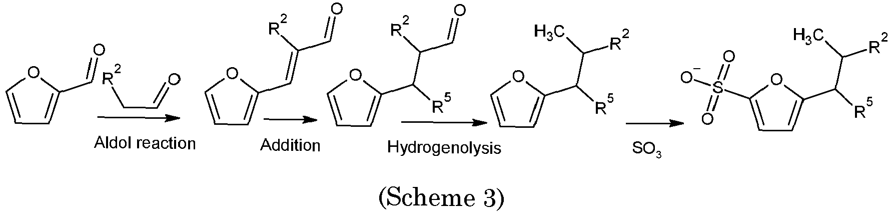

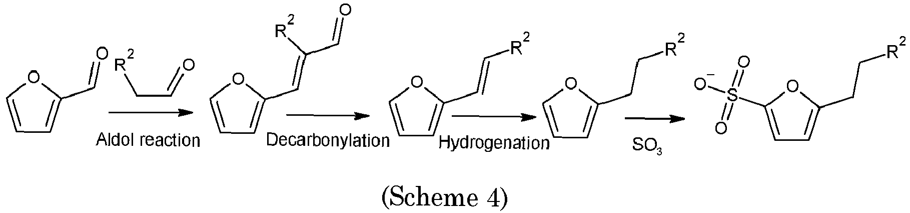

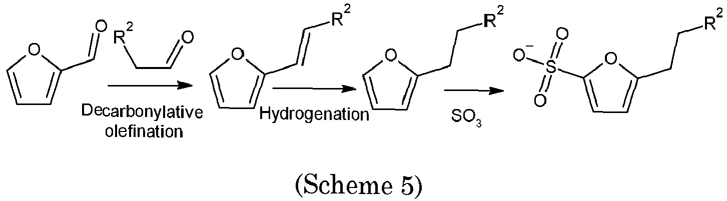

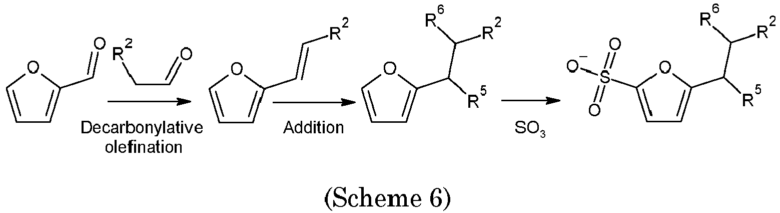

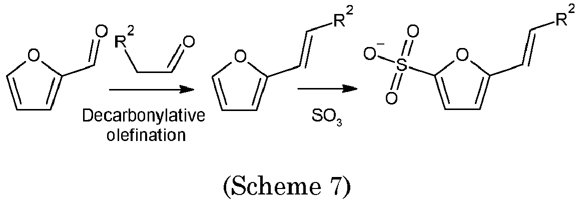

It may be appreciated that Schemes 1-7 are merely illustrative for the specific embodiments wherein furfural, an aldehyde and sulfur trioxide are used. Accordingly, any other reagents as mentioned in this description can be used as a substitute in Schemes 1-7.

The alkyl chains in any of the compounds described herein, illustrated as R², R⁴, R⁵ and R⁶ in the various formulae, may serve as the hydrophobic tail or tails. In general, any of these chains and preferably all of these, independently and individually comprise at least 4 carbon atoms, preferably at least 6 carbon atoms, more preferably between 6 to 26 carbon atoms, most preferably between 6 and 18 carbon atoms. The number of carbon atoms in each chain, the ratio of carbon atoms between the chains, and their position with respect to the furan and the hydrophilic group can suitably be selected depending on the desired properties of the surfactant.

For the purpose of clarity and a concise description, features are described herein as part of the same or separate embodiments, however, it will be appreciated that the scope of the invention may include embodiments having combinations of all or some of the features described.

The invention may further be illustrated by the following nonlimiting examples.

### Example 1

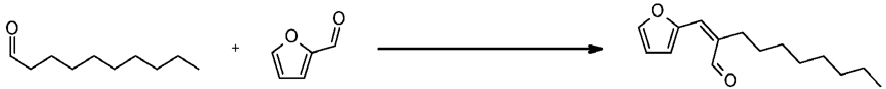

A reactor was charged with potassium hydroxide (0.18 g) and Aliquat-336 (0.84 g, 1.0 ml). Stirring was started intensively then furfural (2.00 g, 1.72 ml) was added dropwise to the mixture. Stirred for 5 minutes. Decanal (3.84 g, 4.62 ml) was added dropwise to the mixture over ~20 minutes. Stirred at room temperature for 24 hours. The mixture was partitioned between ethyl acetate (20 ml) and water (20 ml). The organics were separated and the aqueous extracted with a second portion of ethyl acetate (20 ml). The combined organics were dried (Na₂SO₄), filtered and concentrated to a liquid. This was purified by vacuum distillation to yield the desired α,β-unsaturated aldehyde (3.785 g, 78 %). The structure was confirmed by NMR and GCMS.

### Example 2

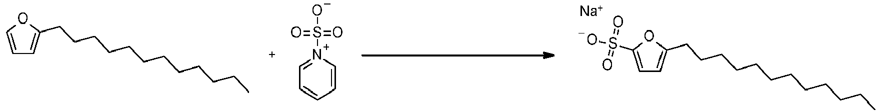

To a reactor was charged sulfur trioxide-pyridine complex (5.8 g) and acetonitrile (25 ml) and the stirring was started. To this slurry was added a solution of 2-dodecylfuran (8.5 g) in acetonitrile (9 ml). Stirred at room temperature for 5 days. The mixture was slowly poured into a stirred reactor containing water (125 ml). Heated to 70°C and held for 1 hour. The organic aqueous phase was separated and the organics extracted with a second portion of water (50 ml). The combined aqueous phase was adjusted to pH7 with solid sodium carbonate. The mixture was concentrated to induce precipitation of a solid. The mixture was cooled to room temperature with stirring and the solid was isolated by filtration, then washed with isopropanol (20 ml). The filtrates were then concentrated further and a further batch of solid began to precipitate, then the mixture was cooled and the solid was isolated by filtration, then washed with isopropanol. The isolated solid was dried under vacuum to yield the desired product (6.68 g, 59 %). The structure was confirmed by NMR and GCMS.

## Claims

1. Method for the preparation of a first furan-based surfactant precursor of formula (I), said method comprising reacting a furanic compound according to formula (V) with a carbonyl compound according to formula (VI), 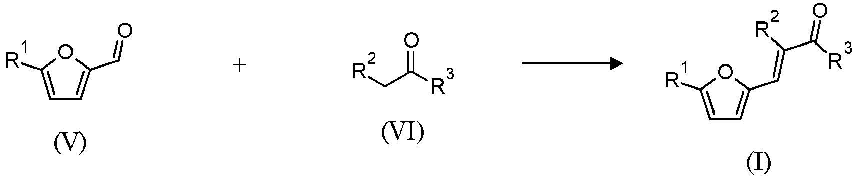
wherein R¹ is H or CH₂OH;
R² is H or an aliphatic group, preferably an aliphatic chain; and
R³ is H, OR⁴ or R⁴, wherein R₄ is H or an aliphatic group, preferably an aliphatic chain.

2. Method for the preparation of the first furan-based surfactant precursor, wherein R³ is H and wherein compound (VI) is prepared by *in situ* oxidition of R²(CH₂)₂OH or by *in situ* reduction of R²-CH₂-CO₂R⁴.

3. Method for the preparation of a second furan-based surfactant precursor of any of formulae (IIa)-(IIc), said method comprising reacting a first furan-based surfactant precursor of formula (I) in, respectively, a decarbonylation reaction wherein R³ is H or a decarboxylation reaction wherein R³ is OR⁴, a hydrogenation and hydrogenolysis, or an addition reaction, 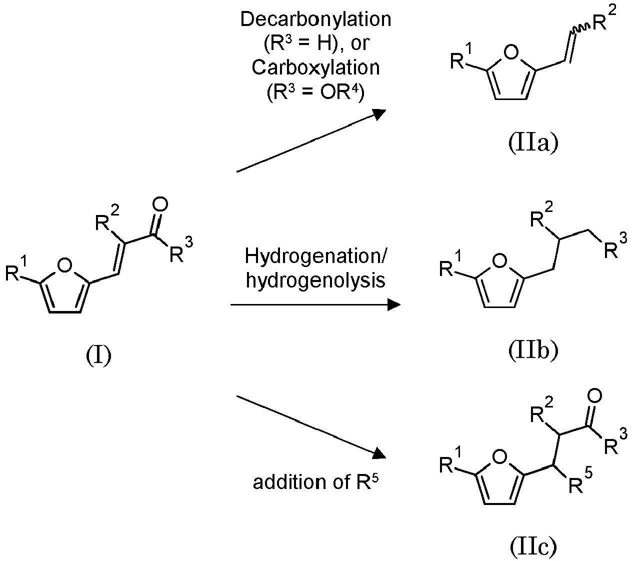
wherein R¹ is H or CH₂OH;
R² is H or an aliphatic group, preferably an aliphatic chain;
R³ is H, OR⁴ or R⁴, wherein R₄ is H or an aliphatic groups, preferably an aliphatic chain; and
wherein R⁵ is an aliphatic group, preferably an aliphatic chain.

4. Method according to the previous claim, wherein the first furan-based surfactant precursor of formula (I) is prepared according to a method according to any of claims 1-2.

5. Method for the preparation of the first furan-based surfactant precursor of formula (IIa), said method comprising, reacting a furanic compound according to formula (V) with a carbonyl compound according to formula (VIa) in a decarbonylative olefination reaction, 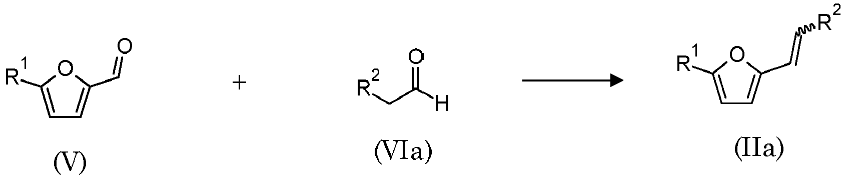
wherein R¹ is H or CH₂OH;
R² is H or an aliphatic group, preferably an aliphatic chain.

6. Method for the preparation of a third furan-based surfactant precursor of any of formulae (IIIa)-(IIId), said method comprising reacting a second furan-based surfactant precursor of, respectively, formula (IIa), (IIa), (IIc) and (IIc) wherein R³ is H, in an addition reaction, a hydrogenation reaction, a decarbonylation reaction, or a hydrogenolysis reaction respectively, 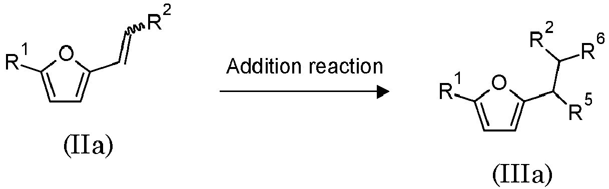 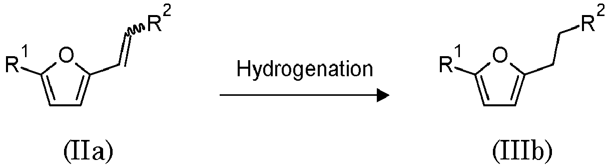 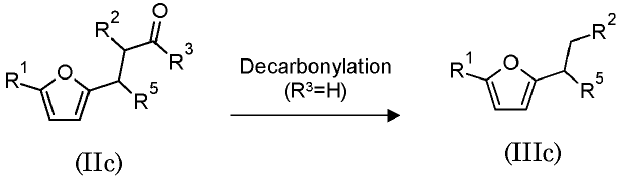 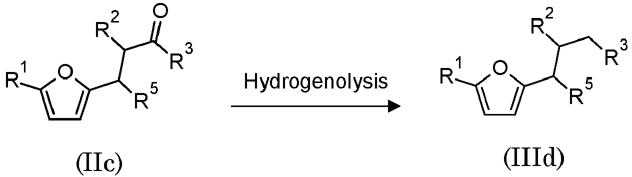
wherein R¹ is H or CH₂OH;
R² is H or an aliphatic group, preferably an aliphatic chain;
R⁵ and R⁶ independently are an aliphatic group, preferably an aliphatic chain, and
wherein ―CH(R⁵)-CH(R²)(R⁶), ―CH₂-CH₂R², ―CH(R⁵)-CH₂(R²), and ―CH(R⁵)-CH(R²)-CH₂R³ of formulae (IIIa)-(IIId) respectively, each represent the hydrophobic tail comprising 6 or more carbon atoms, preferably 6 to 26 carbon atoms, most preferably between 6 and 18 carbon atoms.

7. Method according to the previous claim, wherein the second furan-based surfactant precursor of any of formulae (IIa) and (IIc) is prepared according to a method according to any of claims 3-5.

8. Method for the preparation of a furan-based surfactant or a salt thereof according to any of formula (IVa)-(IVf), said method comprising reacting a first or second furan-based surfactant precursor of any of formulae (IIIa), (IIIb), (IIb), (IIIc), (IIId) and (IIa) respectively, to introduce a hydrophilic group, 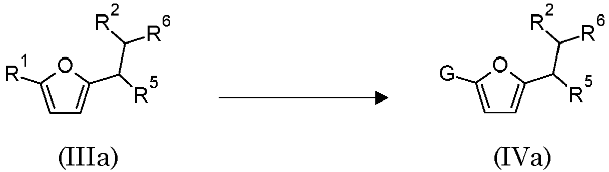 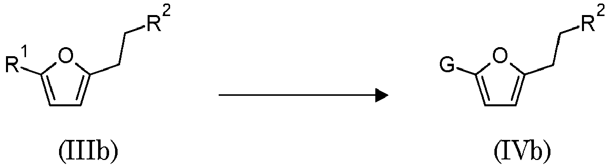 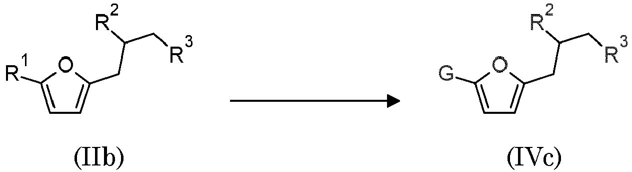 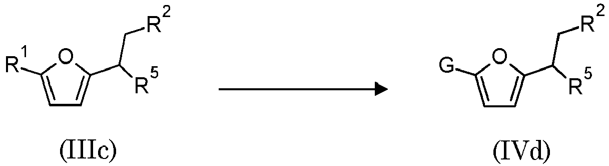 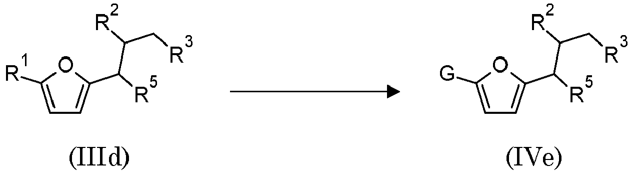 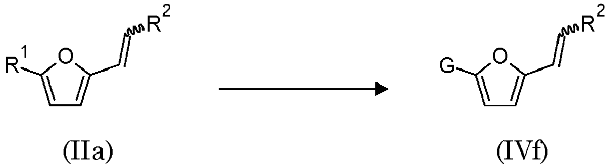
wherein R¹ is H or CH₂OH;
R³ is H, OR⁴ or R⁴;
R² is H or an aliphatic group, preferably an aliphatic chain,
R⁴, R⁵ and R⁶ independently are an aliphatic group, preferably an aliphatic chain; and
wherein G is a moiety comprising a hydrophilic group, and
wherein ―CH(R⁵)-CH(R²)(R⁶), ―CH₂-CH₂R², ―CH₂-CH(R²)-CH₂R³, ―CH(R⁵)-CH₂R², ―CH(R⁵)-CH(R²)-CH₂R³, and ―CH=CHR² of formulae (IIIa), (IIb), (IIIc), (IIId) and (IIa) respectively, as well as of formulae (IVa)-(IVf) respectively, each represent the hydrophobic tail comprising 6 or more carbon atoms, preferably 6 to 26 carbon atoms, most preferably between 6 and 18 carbon atoms.

9. Method according to the previous claim, wherein the first furan-based surfactant precursor of any of formulae (IIa) and (IIb) is prepared according to a method according to any of claims 3-5.

10. Method according to claim 8, wherein the second furan-based surfactant precursor of any of formulae (IIIa)-(IIIc) is prepared according to a method according to any of claims 6-7.

11. Method according to any of claim 8-10, wherein the hydrophilic group is selected from the group consisting of sulfate, sulfonate, sulfinate, thiosulfate, sulfamidate, carboxylate, sarcosinate and taurate, phosphate, pyrophosphate, phosphonate, amines or ammonium, polyammonium, hydroxyammonium, pyridinium, picolinium, imidazolinium, benzimidazolinium, oxonium, sulfonium and phosphonium.

12. Method according to any of claim 8-10, wherein the hydrophilic group comprises non-ionic group, preferably poly(ethylene oxide), poly(co-ethylene oxide co-propylene oxide) (also referred to as poloxamers), polyglycosides, isosorbide and its derivatives, 1,4-sorbitane and its derivatives, and the like.

13. Method according to any of claim 8-11, wherein G is selected from the group consisting of sulfate (-O-SO₃⁻), sulfonate (-SO₃⁻), sulfinate (-SO₂⁻), thiosulfate (-O-S₂O₂⁻), sulfamidate (-NH-SO₃⁻), carboxylate (-CO₂⁻), sarcosinate and taurate (-NR-R-CO₂⁻), phosphate (-O-PO₃⁻ or -O-PO₂-OR⁻), pyrophosphate (-O-PO₂-O-PO₂-OR²⁻), phosphonate (-PO₂R⁻ or -PO₃⁻), amines or ammonium (-NR₃⁺), polyammonium (-NR₂-R-NR₃²⁺), hydroxyammonium (-NR₂OH⁺), pyridinium (-NC₅H₅⁺), picolinium (-NC₅H₅-R⁺), imidazolinium ( 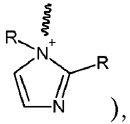 benzimidazolinium 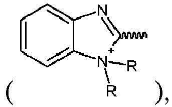 oxonium (-OR₂⁺), sulfonium (-SR₂⁺), phosphonium (-PR₃⁺), methylene sulfate (-CH₂O-SO₃⁻), methylene sulfonate (-CH₂SO₃⁻), methylene sulfinate (-CH₂SO₂⁻), methylene thiosulfate (-CH₂O-S₂O₂⁻), methylene sulfamidate (-CH₂NH-SO₃⁻), methylene carboxylate (-CH₂CO₂⁻), methylene sarcosinate and taurate (-CH₂NR-R-CO₂⁻ ), methylene phosphate (-CH₂O-PO₃⁻ or - CH₂O-PO₂-OR), methylene pyrophosphate (-CH₂O-PO₂-O-PO₂-OR²), methylene phosphonate (-CH₂PO₂R⁻ or - CH₂PO₃⁻), methylene amines or ammonium (-CH₂NR₃⁺), methylene polyammonium (-CH₂NR₂-R-NR₃²⁺), methylene hydroxyammonium (-CH₂NR₂OH⁺), methylene pyridinium (-CH₂NC₅H₅⁺), methylene picolinium (-CH₂NC₅H₅-R⁺), methylene imidazolinium 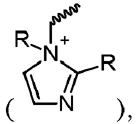 methylene benzimidazolinium 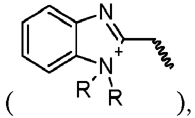 methylene oxonium (-CH₂OR₂⁺), methylene sulfonium (-CH₂SR₂⁺), and methylene phosphonium (-CH₂PR₃⁺) wherein R is H or a C₁-C₃ alkyl, preferably H or Me, preferably wherein G is carboxylate or sulfonate.

14. Method according to any of the previous claims, wherein R¹ is H and/or wherein R³ is H.

15. Method according to any of the previous claims, wherein any of R², R⁴, R⁵ and R⁶, independently comprise an aliphatic chain comprising at least 4 carbon atoms, preferably at least 6 carbon atoms, more preferably between 6 to 26 carbon atoms, most preferably between 6 and 18 carbon atoms.
